# EUROPÄISCHE PATENTSCHRIFT

(11) **EP 1 489 966 B1**
(45) Veröffentlichungstag und Bekanntmachung des Hinweises auf die Patenterteilung: **08.11.2006**
(21) Anmeldenummer: 03720396.5
(22) Anmeldetag: 31.03.2003
(51) Int. Cl.: A61B 5/055, A61B 5/11

(54) **PASSIVBEWEGUNG EINES PATIENTEN IN EINEM MAGNET-RESONANZ-TOMOGRAPHEN**
PASSIVE MOVEMENT OF A PATIENT IN A MAGNETIC RESONANCE TOMOGRAPH
MOUVEMENT PASSIF D'UN PATIENT DANS UN TOMOGRAPHE A RESONANCE MAGNETIQUE

(30) Priorität: 01.04.2002 DE 10214798; 06.08.2002 DE 10235963
(43) Veröffentlichungstag der Anmeldung: 29.12.2004
(73) Patentinhaber: Elias, Ilan, 60439 Frankfurt am Main (DE)
(72) Erfinder: Elias, Ilan, 60439 Frankfurt am Main (DE)
(74) Vertreter: KEIL & SCHAAFHAUSEN Patentanwälte
(86) Internationale Anmeldenummer: PCT/EP2003/003337
(87) Internationale Veröffentlichungsnummer: WO 2003/082107

(56) Entgegenhaltungen:
- US-A- 5 743 264
- US-A- 5 772 595

## Beschreibung

Die Erfindung betrifft ein Verfahren zum Erzeugen von (Bild-) Aufnahmen sowie eine Vorrichtung zum Erzeugen einer Passivbewegung eines Körperteils eines Patienten oder von Gegenständen, bspw. Instrumenten, in einem Magnet-Resonanz-Tomographen mit einer Auflage zur Lagerung von zumindest einem Körperteil eines Patienten, die gemeinsam mit einer Patientenliege oder dgl. in den Kanal eines Magnet-Resonanz-Tomographen einfahrbar ist.

Zur biomechanischen Untersuchung von z. B. menschlichen oder tierischen Gelenken oder Pflanzen werden u. a. auch Magnet-Resonanz-Tomographen (MRT) eingesetzt, die auch eine Darstellung weicher Strukturen ermöglichen. So kann der Bewegungsablauf eines (gesunden) Gelenks mit Sehnen, Knorpeln und dgl. mit einem derartigen Verfahren erforscht werden. Auch in der Materialprüfung und bei der Erprobung des Werkstoffverhaltens von verschiedenen Gegenständen werden häufig Untersuchungen mit Magnet-Resonanz-Tomographen durchgeführt.

Auch um eine optimale Diagnose von Verletzungen und Erkrankungen von Gelenken, wie bspw. des Sprunggelenkes, zu ermöglichen, ist es bekannt, einen Patienten oder zumindest relevante Körperteile mit einem Magnet-Resonanz-Tomographen aufzunehmen und anhand der erstellten Aufnahmen eine Diagnose zu stellen. Hierzu ist es häufig erforderlich, das betroffene Körperteil je nach Bedarf verschiedenartig und definiert zu lagern, bzw. bewegen zu können.

Bislang werden außer statischen Momentaufnahmen daher statische Bilder in verschiedenen Stellungen erzeugt, wobei die Positionen manuell über eine Mechanik eingestellt werden, und die Betrachtung der resultierenden Bilder durch eine Bildschleife (cine-mode) erfolgt. Dieses bspw. aus der US 5,541,515 und der US 5,899,859 bekannte Verfahren ist jedoch besonders zeitaufwendig und kann daher nur in Einzelfällen angewandt werden, so dass die Möglichkeiten eines modernen Magnet-Resonanz-Tomographen, der auch sehr schnelle Bildaufnahmen ermöglicht, nicht ausgenutzt werden. Somit werden die Untersuchungsmöglichkeiten von Materialien, Gegenständen oder Körperteilen mit einem Magnet-Resonanz-Tomographen, die sowohl die knöchernen und knorpeligen Anteile des menschlichen Körpers als auch Weichteilstrukturen exzellent darstellen kann, nicht optimal eingesetzt.

Die US 5,772,595 offenbart einen Magnet-Resonanz-Tomographen mit einstellbaren Befestigungsmitteln, die die Kontrolle der Bewegung und der Position der Gelenke ermöglichen, um die Funktionalität der Gelenke durch eine Reihe von Aufnahmen zu schätzen. Während der Aufnahmen bleibt der untersuchte Körperteil stationär.

Zudem besteht der Nachteil, dass durch statische Momentaufnahmen nicht alle Schäden an Gegenständen oder Erkrankungen bzw. Verletzungen am menschlichen Körper detektieren lassen, insbesondere, da es bisher nicht möglich ist, Aufnahmen in Bewegung in hinreichender Qualität zu erstellen. Dies beeinträchtigt jedoch die exakte Materialforschung sowie die klinische Diagnostik, da somit die Wahrscheinlichkeit (Sensitivität) einen vorhandenen Schaden bzw. pathologischen Befund bei diversen Strukturen zu ermitteln, noch nicht optimal bzw. teilweise nicht möglich ist. Im Gegensatz zur Röntgenuntersuchung gibt es nämlich in der Magnet-Resonanz-Tomographie bisher keine zuvor fest definierten und reproduzierbaren Einstellungen für Darstellungsmöglichkeit von Echtzeitbewegungen.

Dies liegt insbesondere daran, dass aufgrund des starken Magnetfeldes in einem Magnet-Resonanz-Tomographen die Verwendung herkömmlicher Bewegungsapparate nicht möglich ist. Herkömmliche elektromechanische Motoren, die üblicherweise in Bewegungsapparaten eingesetzt werden, bestehen aus Magneten und Spulen, die in den hohen Magnetfeldern (0,2 - 3 Tesla) eines Magnet-Resonanz-Tomographen besonders stark deflektieren und damit zu

Bildverzerrungen, sog. Bildartefakten, führen. Sobald derartige Bildartefakte auftreten, ist eine sachgemäße Untersuchung jedoch nicht mehr möglich.

Aufgabe der Erfindung ist es daher, ein Verfahren zum Erzeugen von Aufnahmen während einer Passivbewegung eines Gegenstands, d. h. Echtzeitaufnahmen, sowie eine Vorrichtung der eingangs genannten Art bereitzustellen, die eine definierte reproduzierbare Passivbewegung eines Körperteils oder Gegenstands in einem Magent-Resonanz-Tomographen ermöglicht, ohne dabei Bildverzerrungen, d. h. Bildartefakte, zu erzeugen, die eine Auswertung unmöglich machen.

Diese Aufgabe wird erfindungsgemäß im Wesentlichen durch das Verfahren gemäß Anspruch 1 sowie durch die Vorrichtung gemäß Anspruch 2 gelöst. Dabei werden die (Bild-) Aufnahmen in Echtzeit während der Passivbewegung von Gegenständen erzeugt werden, ohne dass hierbei Artefakte auftreten. Die Aufnahmen selbst werden dabei deutlich schneller erstellt als bei der herkömmlichen Erzeugung einer Folge von Einzelaufnahmen, zwischen denen jeweils der Gegenstand bewegt wird. Als Ergebnis dieses Verfahren erhält man eine Serie von Bildern, d. h. einen Film, der den untersuchten Gegenstand nicht in Ruhe sondern während der Bewegung zeigt. Hierzu werden von einem Techniker gleichzeitig mit der Erzeugung der definierten Passivbewegung Aufnahmen von menschlichen oder tierischen Körperteilen, von Pflanzen oder Gegenständen aus anderen Materialien, wie Instrumenten, gemacht, die dann bspw. von einem Materialprüfer, Biomechaniker oder in Kombination mit diagnostischen Untersuchungsergebnissen von einem Arzt ausgewertet werden können. Einige biomechanische Vorgänge lassen sich nur bei der Untersuchung dieser tatsächlichen Bewegungsabläufe in z. B. einem Gelenk erklären, was bei der bisher bekannten Aneinanderreihung statischer Aufnahmen im sog. "cine-mode" nicht möglich ist. Auch in der Materialforschung ist bspw. die Risseinleitung und der Rissfortschritt in Materialien ein dynamischer Vorgang, der häufig nur durch die erfindungsgemäßen Echtzeitaufnahmen bewertet werden kann, nicht jedoch in statischen Aufnahmen.

Weiter wird die Aufgabe der Erfindung mit einer Vorrichtung im Wesentlichen dadurch gelöst, dass die Auflage motorisch angetrieben innerhalb des Kanals des Magnet-Resonanz-Tomographen um wenigstens eine Achse verschwenkbar ist und dass die innerhalb des Kanals des Magnet-Resonanz-Tomographen liegenden Komponenten der Auflage und ihres Antriebs aus nicht-ferromagnetischen Materialien bestehen. Durch diese Ausbildung der Vorrichtung wird die Sensitivität der Magnet-Resonanz-Untersuchung bspw. im Bereich der Gelenkuntersuchung deutlich erhöht, indem vordefinierte kontinuierlichen Bewegungen eingestellt und die Bewegungen reproduziert werden können, um Echtzeitaufnahmen (Online- bzw. Realtime-Aufnahmen) darstellen zu können. Die Vorrichtung ermöglicht es dabei, ein Körperteil im Magnet-Resonanz-Tomographen in vollem Bewegungsumfang passiv und der jeweiligen Untersuchungssituation wunschgemäß zu bewegen. Durch die damit verbesserten und erweiterten Materialprüfungs- und Untersuchungsmöglichkeiten lassen sich Schäden und vermutete Verletzungen und dgl. weit besser als bisher feststellen und verifizieren. Durch die Ausbildung der in dem Kanal des Magnet-Resonanz-Tomographen eingesetzten Komponenten aus nicht-ferromagnetischen Materialien wird zudem das Auftreten von Bildartefakten, die eine Untersuchung erschweren, reduziert. Mit dieser Vorrichtung lassen sich auch Instrumente in dem Kanal des Magnet-Resonanz-Tomographen bewegen, so dass nicht nur eine Untersuchung sondern auch eine gleichzeitige Bearbeitung oder Behandlung der untersuchten Gegenstände möglich ist. Zur Vermeidung von Bildartefakten müssen diese Instrumente mit Magnet-Resonanz-Tomographen kompatibel sein, d. h. aus einem geeigneten Material bestehen.

Es wurde herausgefunden, dass das Auftreten von Bildartefakten dadurch weiter unterbunden werden kann, dass der Antrieb zum Verschwenken der Auflage mittels eines Piezoelektromotors erfolgt.

Vorzugsweise ist der Antrieb zum Verschwenken der Auflage über eine Steuereinheit bzw. Control Unit gesteuert, die geerdet und gegenüber magnetischer Strahlung abgeschirmt ist. Mit der erfindungsgemäßen Vorrichtung lassen sich die Bewegungen daher elektronisch und automatisch kontrolliert von außerhalb des Magnet-Resonanz-Tomographen-Raumes in immer denselben exakt reproduzierbaren Positionen einstellen. Dabei kann die Lage zwischen dem zu untersuchenden Körperteil und dem Magnet-Resonanz-Tomographen während des gesamten diagnostischen Vorgangs unverändert bleiben. Gleichzeitig können jedoch erstmals durch Motoren gesteuerte genau definierte Bewegungen während des Aufnahmevorgangs vorgenommen werden. Dies ermöglicht dem Untersuchenden zum Einen eine bestimmte Aufnahme gezielt zu erstellen, zum Anderen aber auch die Bewegung selbst im Sinne einer Echtzeitaufnahme darzustellen. Neben der Verwendung eines Piezoelektromotors zum Antrieb der Auflage ist es auch möglich, hierzu pneumatische oder hydraulische Antriebe einzusetzen.

Wenn die Steuereinheit außerhalb der Zone um den Magnet-Resonanz-Tomographen angeordnet ist, in welcher im Betrieb die magnetische Flußdichte größer oder gleich 0,2 Tesla ist, wird die Funktion der Steuereinheit durch das starke Magnetfeld des Magnet-Resonanz-Tomographen nicht beeinträchtigt. Gleichzeitig können durch die Steuereinheit verursachte Bildartefakte vermieden werden.

Die Steuerung der Auflage kann dadurch weiter verbessert werden, dass die Steuereinheit mit wenigstens einem Sensor, insbesondere mit einem optischen Encoder, zur Erfassung der Position der Auflage bzw. der Motoren versehen ist.

Um das Auftreten von die Diagnostik erschwerenden Bildartefakten noch wirkungsvoller vermeiden zu können, kann die Steuereinheit mit dem Antrieb der Auflage und ggf. mit den Sensoren über geerdete und abgeschirmte Leitungen verbunden sein, welche außerhalb des Kanals des Magnet-Resonanz-Tomographen mit Ferriten versehen sind.

In Weiterbildung des Erfindungsgedankens ist es vorgesehen, dass die Auflage um zwei Achsen unabhängig von einander motorisch angetrieben verschwenkbar ist. Damit lassen sich die physiologischen Bewegungsabläufe der zu untersuchenden Körperteile noch besser darstellen.

Der physiologische Bewegungsablauf eines Sprunggelenks lässt sich mit der erfindungsgemäßen Vorrichtung dann besonders gut nachbilden, wenn die Auflage um eine erste horizontale Achse und eine zweite gegenüber der vertikalen um etwa 35° in der Horizontalebene und um etwa 18° in der Sagittalebene geneigte zweite Achse verschwenkbar ist. Diese Neigung der zweiten Achse entspricht der von Van den Bogard ermittelten durchschnittlichen geometrischen Achse des unteren Sprunggelenks.

Die bspw. beim Laufen auf das Sprunggelenk etc. einwirkenden Druckkräfte können während der Untersuchung in dem Magnet-Resonanz-Tomographen dadurch nachgebildet werden, dass Mittel zum Fixieren des zumindest einen Körperteils des Patienten auf der Auflage vorgesehen sind und dass die Auflage wenigstens bereichsweise relativ zu den Mitteln zum Fixieren bewegbar ist.

Vorzugsweise ist die Auflage dabei pneumatisch oder hydraulisch relativ zu den Mitteln zum Fixieren des Körperteils bewegbar. Auf diese Weise kann eine stufenweise Komprimierung des zu untersuchenden Körperteils stattfinden, die ebenfalls zu einer Änderung der Konfiguration der Einzelteile des Körpers führt, die den Belastungen bspw. beim Laufen oder dgl. nachgebildet sind.

Die Erzeugung von Echtzeitaufnahmen eines Gegenstandes ist mit der erfindungsgemäßen Vorrichtung möglich, wenn diese derart mit dem Magent-Resonanz-Tomographen gekoppelt ist, dass die (Bild-) Aufnahmen gleichzeitig mit der Erzeugung der Passivbewegung durchgeführt werden.

Im Folgenden wird die Erfindung anhand einer Ausführungsform und unter Bezugnahme auf die beigefügte Zeichnung näher erläutert. Es zeigen:
- Fig. 1: einen Längsschnitt durch den Kanal eines Magent-Resonanz-Tomographen mit einer auf einer Patientenliege angeordneten er findungsgemäßen Vorrichtung,

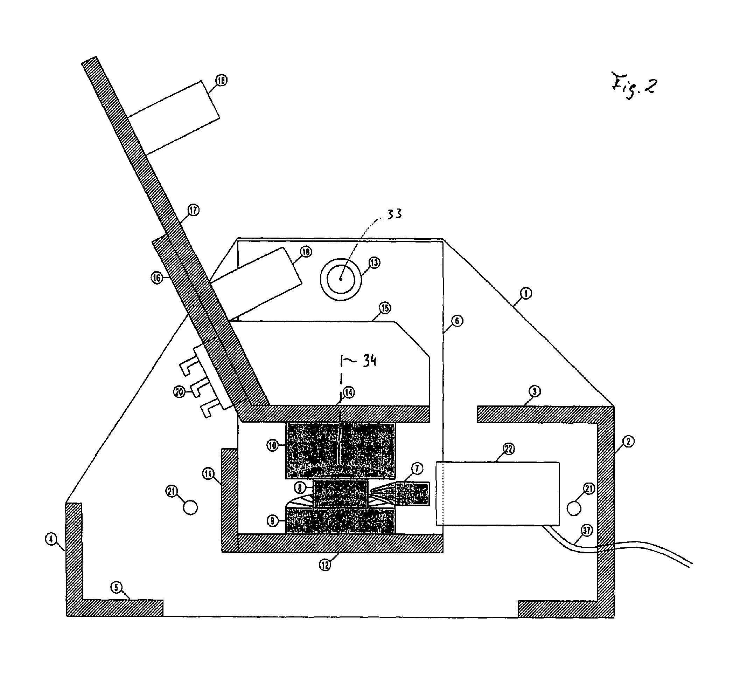

- Fig. 2: eine Schnittansicht der Vorrichtung senkrecht zu der Schnittebene von Fig. 1 und

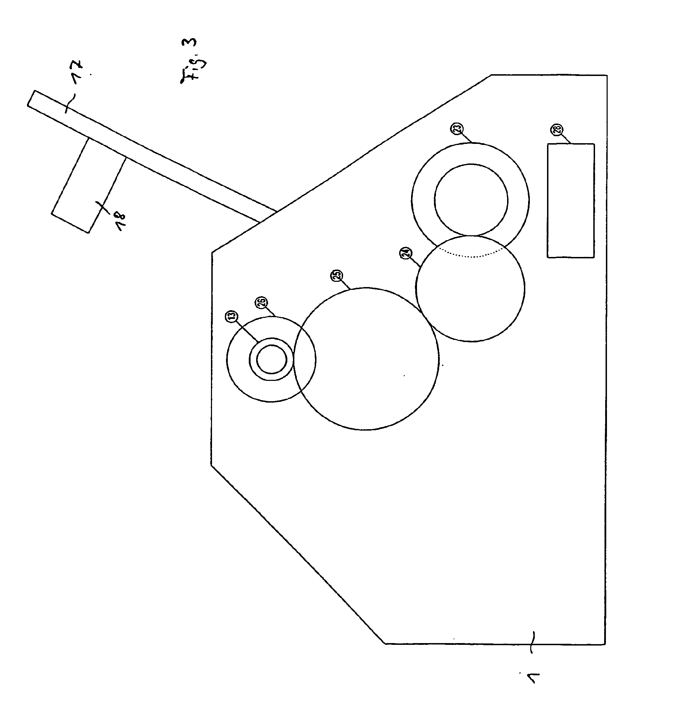

- Fig. 3: eine Seitenansicht der Vorrichtung nach Fig. 2.

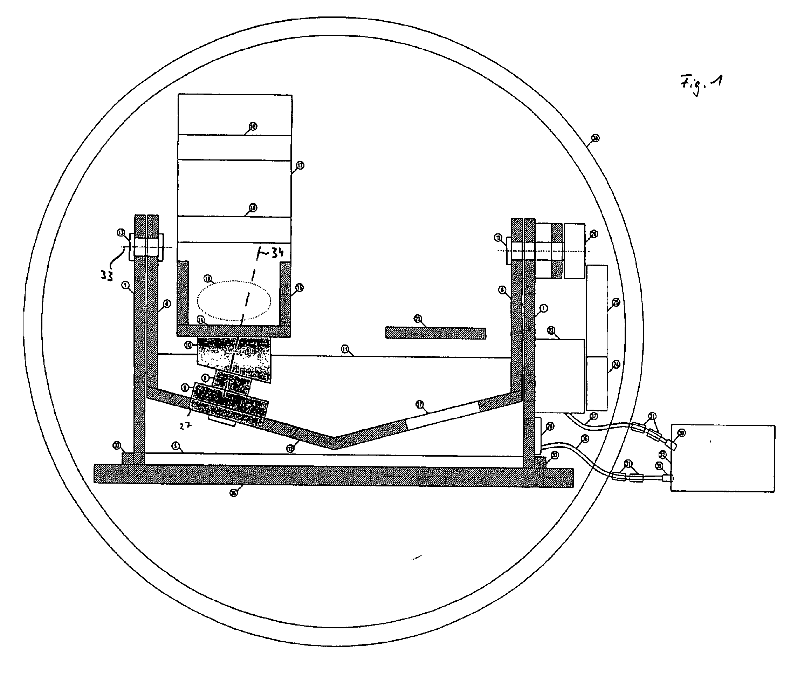

Die in den Figuren dargestellte Vorrichtung ist zum Einsatz in einem Magnet-Resonanz-Tomographen (MRT) bspw. mit einer magnetische Flußdichte zwischen 0,2 und 3,0 Tesla vorgesehen. Der Magnet-Resonanz-Tomograph ist in Fig. 1 durch seinen Kanal 38 angedeutet. In der gezeigten Ausführungsform ist die Vorrichtung zur Untersuchung des Sprunggelenks eingerichtet. Es ist jedoch möglich, mit dieser Vorrichtung andere Körperteile oder allgemein Gegenstände aus beliebigen Materialien zu untersuchen. Die Vorrichtung weist dabei eine Auflage 14 für die Ferse des Fußes und eine dazu abgewinkelt verlaufende Auflage 17 für die Fußsohle auf. Die Innen- und Außenknöchel des Fußes werden durch Seitenwände 15 stabilisiert, während die Auflage 17 für die Fußsohle auf einer Rückwand 16 gehalten ist. In Fig. 1 ist der zu untersuchende Fuß schematisch durch Bezugsziffer 19 angedeutet.

Die Vorrichtung ist, wie in Fig. 1 gezeigt, mittels einer angedeuteten Verbindungseinheit 30 auf einer Patientenliege 35 befestigt. Ein Patient kann dabei auf dem Rücken liegend mit der Patientenliege 35 in den Kanal 38 des Magnet-Resonanz-Tomographen eingeschoben werden, wobei die Vorrichtung an einem Ende der Patientenliege 35 angeordnet ist. Mit Schnallen 18 kann der Fuß dabei in der Vorrichtung fixiert gehalten werden. Der nicht zu untersuchende Fuß kann auf einer weiteren Auflage 29 abgelegt werden und wird bei der Untersuchung nicht miterfasst.

Die auf der Patientenliege 35 angeordnete Vorrichtung umfasst weiter zwei vertikale Seitenwände 1 und eine vertikale Frontwand 2, die in eine horizontale Frontwand 3 übergeht. Auf der der vertikalen Frontwand 2 gegenüberliegenden Seite ist eine vertikale Rückwand 4 vorgesehen, die mit einer unteren horizontalen Rückwand 5 verbunden ist. Parallel zu den Seitenwänden 1 verläuft eine weitere vertikale Wand 6, die über Kugellager 13 um eine horizontale Achse 33 schwenkbar an den Seitenwänden 1 angelenkt ist. Die vertikalen Wände 6 sind dabei über eine Rückwand 11 und eine V-förmige Bodenwand 12 miteinander verbunden.

In der V-förmigen Bodenwand 12 sind zwei Aussparungen 27 vorgesehen, in welcher eine Befestigungseinheit 9 aufnehmbar ist. Über ein Zwischenteil 8 trägt die Befestigungseinheit 9 eine weitere Befestigungseinheit 10 mit einem Zahnrad, auf der die Auflage 14 für die Ferse sowie die Rückwand 16 mit der Auflage 17 für die Fußsohle angebracht ist. Die Auflage 14 für die Ferse sowie die Rückwand 16 mit der Auflage 17 für die Fußsohle sind dabei über die Befestigungseinheit 10 relativ zu der Befestigungseinheit 9 verdrehbar, wobei durch die Neigung der V-förmigen Bodenwand 12 und die entsprechende Ausgestaltung der Befestigungseinheiten 9 und 10 die Auflage 14 für die Ferse mit der Rückwand 16 und der Auflage 17 für die Fußsohle um eine um etwa 35° in der Horizontalebene und um etwa 18° in der Sagittalebene gegenüber der Vertikalen geneigten Achse 34 verdrehbar sind.

Den Befestigungseinheiten 9 und 10 ist ein Piezoelektromotor 22 zugeordnet, der ein Zahnrad 7 mit konischer Spitze zur Bewegung der Befestigungseinheit 10 relativ zu der Befestigungseinheit 9 trägt. In gleicher Weise ist an der vertikalen Seitenwand 1 ein Piezoelektromotor 23 vorgesehen, welcher ein erstes Zahnrad 24 trägt, das über ein weiteres Zahnrad 25 mit einem Zahnrad 26 in Eingriff bringbar ist, das drehfest mit einer der Seitenwände 6 verbunden ist. Auf diese Weise lassen sich die Seitenwände 6 relativ zu den Seitenwänden 1 durch den Motor 23 angetrieben verschwenken.

An der Rückwand 16 ist eine Druckventileinheit 20 angeordnet, mit welcher die Auflage 17 für die Fußsohle relativ zu der Rückwand 16 verschoben werden kann. Auf diese Weise kann stufenlos Druck auf die Fußsohle erzeugt werden.

In einer der Seitenwände 1 sind zwei optische Encoder 21 positioniert, welche die Position der Seitenwände 6 relativ zu den Seitenwänden 1 erfassen können. Die durch die Encoder 21 ermittelten Daten können über nicht dargestellte Leitungen an eine Elektrobox 28 weitergeleitet werden, die wiederum über ein abgeschirmtes Kabel 36 mit einer Steuereinheit 32 (Control Unit) verbunden ist. Von der Steuereinheit 32 werden auch die Motoren 22 und 23 über abgeschirmte Kabel 37 angesteuert. Außerhalb des Kanals 38 des Magnet-Resonanz-Tomographen sind auf den abgeschirmten Leitungen 36 und 37 Ferritkeme 31 angeordnet. Die Verbindung der Leitungen 36 und 37 mit der Steuereinheit 32 erfolgt dabei über ebenfalls abgeschirmte Kabelstecker 39.

Die für die Vorrichtung verwendeten Materialien werden dabei vom Magnetfeld des Magnet-Resonanz-Tomographen nicht beeinflusst und erzeugen daher keine Bildartefakte, die eine Diagnostik unmöglich machen, wenn für die innerhalb des Kanals 38 des Magnet-Resonanz-Tomographen angeordneten Komponenten nicht-ferromagnetische Materialien verwendet werden. Dies sind bspw. VA4-Edelstahlschrauben und -gewinde, Aluminiumplatten, Stifte, Schrauben und Luftdruckdüsen aus Messing, Kunststoffschrauben, und Glas- und Kermamikkugellager. Dabei ist der Einsatz von Polyoxymethylen-Halbwerkzeugen (POM) besonders günstig, da dieser Kunststoff das Hochfrequenzfeld (HF) absorbiert und daher keine störende Strahlung erzeugt.

Die gezeigte Ausführungsform der Vorrichtung zum Erzeugen einer Passivbewegung ist speziell für die Untersuchung von Sprunggelenken eingerichtet. Dabei können durch die Motoren 22 und 23 die Auflage 14 für die Ferse sowie die mit der Rückwand 16 verbundene Auflage 17 für die Fußsohle derart verdreht werden, dass die physiologische Bewegung des Sprunggelenks nachgebildet wird. Durch die Verschiebung der Auflage 17 für die Fußsohle relativ zu der Rückwand 16 mittels der Druckventileinheit 20 kann zudem eine Gewichtsbelastung des Fußes imitiert werden.

Der Magent-Resonanz-Tomograph ist dabei derart mit dem Antrieb der Vorrichtung verbunden, dass die Aufnahmen während der Erzeugung der Passivbewegung erstellt werden können. Auf diese Weise ist es möglich, sowohl kinematische (Echtzeit-)Aufnahmen während der Passivbewegung des Körperteils als auch statische Aufnahmen aus unterschiedlichen Positionen innerhalb des Kanals 38 des Magnet-Resonanz-Tomographen für die Forschung und die klinische Routinediagnostik anzufertigen. Diese Echtzeitaufnahmen des Bewegungsablaufs erweitern dadurch die Einsatzmöglichkeiten des an sich bekannten Magnet-Resonanz-Tomographen erheblich.

Mit der Vorrichtung lassen sich alternativ oder zusätzlich zu der Passivbewegung von Körperteilen eines Patienten auch Instrumente motorisch, d..h. passiv, antreiben, die aus einem mit Magnet-Resonanz-Tomographen kompatiblen Material bestehen, so dass sie keine störenden Bildartefakte erzeugen.

### Bezugszeichenliste:

- 1: Seitenwand
- 2: vertikale Frontwand
- 3: horizontale Frontwand
- 4: vertikale Rückwand
- 5: horizontale Rückwand
- 6: Seitenwand
- 7: Zahnrad mit konischer Spitze
- 8: Zwischenteil
- 9: Befestigungseinheit
- 10: Befestigungseinheit
- 11: Rückwand
- 12: Bodenwand
- 13: Kugellager
- 14: Auflage (Ferse)
- 15: Seitenwand
- 16: Rückwand
- 17: Auflage (Fußsohle)
- 18: Schnalle
- 19: Fuß
- 20: Druckventileinheit
- 21: optische Encoder
- 22: Motor
- 23: Motor
- 24: Zahnrad
- 25: Zahnrad
- 26: Zahnrad
- 27: Aussparung
- 28: Elektrobox
- 29: Auflage
- 30: Verbindungseinheit
- 31: Ferritkerne
- 32: Steuereinheit
- 33: horizontale Achse-x
- 34: vertikale Achse-y
- 35: Patientenliege
- 36: abgeschirmte Elektrokabel
- 37: abgeschirmte Motorkabel
- 38: MRT Kanal
- 39: Kabelstecker

## Patentansprüche

1. Verfahren zum Erzeugen von Aufnahmen einer Passivbewegung eines Gegenstands in einem Magnet-Resonanz-Tomographen, das die folgenden Schritte umfasst:
- Fixieren des Gegenstands auf einer Auflage (14, 17) oder dgl.,
- Erzeugen einer definierten Passivbewegung des Gegenstands durch einen motorischen Antrieb der Auflage (14, 17) innerhalb des Kanals (38) eines Magnet-Resonanz-Tomographen und
- Erzeugen einer Serie von Bild-Aufnahmen in Echtzeit während der Passivbewegung des Gegenstands.

2. Vorrichtung zum Erzeugen einer Passivbewegung eines Körperteils eines Patienten und/oder von mit Magnet-Resonanz-Tomographen kompatiblen Instrumenten oder Gegenständen in einem Magnet-Resonanz-Tomographen in einem Verfahren nach Anspruch 1 mit einer Auflage (14, 17) zur Lagerung von zumindest einem Körperteil (19) eines Patienten, die gemeinsam mit einer Patientenliege (35) oder dgl. in den Kanal (38) eines Magnet-Resonanz-Tomographen einfahrbar ist, wobei die Auflage (14, 17) motorisch angetrieben innerhalb des Kanals (38) des Magnet-Resonanz-Tomographen um wenigstens eine Achse (33, 34) verschwenkbar ist und die innerhalb des Kanals (38) des Magnet-Resonanz-Tomographen liegenden Komponenten der Auflage (14, 17) und ihres Antriebs aus nichtferromagnetischen Materialien bestehen, **dadurch gekennzeichnet, dass** der Antrieb zum Verschwenken der Auflage (14, 17) über eine Steuereinheit (32) oder Control Unit derart gesteuert wird, dass in dem Magnet-Resonanz-Tomographen eine Serie von Bildaufnahmen in Echtzeit während der Passivbewegung erzeugt wird.

3. Vorrichtung nach Anspruch 2, **dadurch gekennzeichnet, dass** der Antrieb zum Verschwenken der Auflage (14, 17) mittels wenigstens eines Piezoelektromotors (22, 23) erfolgt.

4. Vorrichtung nach Anspruch 2 oder 3, **dadurch gekennzeichnet, dass** die Steuereinheit (32) bzw. Control Unit geerdet und gegenüber magnetischer Strahlung abgeschirmt ist.

5. Vorrichtung nach Anspruch 4, **dadurch gekennzeichnet, dass** die Steuereinheit (32) außerhalb der Zone um den Magnet-Resonanz-Tomographen angeordnet ist, in welcher im Betrieb die magnetische Flußdichte größer oder gleich 0,2 Tesla ist.

6. Vorrichtung nach einem der Ansprüche 4 oder 5, **dadurch gekennzeichnet, dass** die Steuereinheit (32) mit wenigstens einem Sensor, insbesondere mit einem optischen Encoder (21), zur Erfassung der Position der Auflage (14, 17) versehen ist.

7. Vorrichtung nach einem der Ansprüche 4 bis 6, **dadurch gekennzeichnet, dass** die Steuereinheit (32) mit dem Antrieb (22, 23) der Auflage (14, 17) und ggf. mit den Sensoren (21) über geerdete und abgeschirmte Leitungen (36, 37) verbunden ist, welche außerhalb des Kanals (38) des Magnet-Resonanz-Tomographen mit Ferriten (31) versehen sind.

8. Vorrichtung nach einem der Ansprüche 2 bis 7, **dadurch gekennzeichnet, dass** die Auflage (14, 17) um zwei Achsen (33, 34) unabhängig voneinander motorisch angetrieben verschwenkbar ist.

9. Vorrichtung nach Anspruch 8, **dadurch gekennzeichnet, dass** die Auflage (14, 17) um eine erste horizontale Achse (33) und um eine gegenüber der Vertikalen um etwa 35° in der Horizontalebene und um etwa 18° in der Sagittalebene geneigte zweite Achse (34) verschwenkbar ist.

10. Vorrichtung nach einem der Ansprüche 2 bis 9, **dadurch gekennzeichnet, dass** Mittel (18) zum Fixieren zumindest eines Körperteils (19) eines Patienten auf der Auflage (14, 17) vorgesehen ist und dass die Auflage (17) wenigstens bereichsweise relativ zu den Mitteln (18) zum Fixieren bewegbar ist.

11. Vorrichtung nach Anspruch 9, **dadurch gekennzeichnet, dass** die Auflage (17) pneumatisch oder hydraulisch relativ zu den Mitteln (18) zum Fixieren des Körperteils (19) bewegbar ist.

12. Vorrichtung nach einem der Ansprüche 2 bis 11, **dadurch gekennzeichnet, dass** der motorische Antrieb der Auflage (14, 17) bspw. über eine Steuerung derart mit dem Magent-Resonanz-Tomographen verbunden ist, dass von dem Magent-Resonanz-Tomographen Aufnahmen gleichzeitig mit der Erzeugung der Passivbewegung des Körperteils eines Patienten und/oder von Instrumenten oder Gegenständen erstellt werden.

## Claims

1. A method of taking pictures of a passive movement of an object in a magnetic resonance tomograph comprising the following steps:
- fixing of the object on a support(14, 17) or the like;
- generating a defined passive movement of the object by a motorized drive of the support (14,17) within the duct (38) of a magnetic resonance tomograph; and
- taking a series of pictures in real time during the passive movement of the object.

2. An apparatus for generating a passive movement of a part of a patient's body and/or of instruments or objects compatible with magnetic resonance tomographs within a magnetic resonance tomograph in a process according to claim 1, comprising a support (14,17) for positioning at least one part of a patient's body, which together with a patient's bed (35) or the like is movable into the duct (38) of a magnetic resonance tomograph , with the support (14, 17) which is driven by a motor within the duct (38)of the magnetic resonance tomograph, being pivotable about at least one axis (33, 34), and with the components of the support (14, 17) disposed within the duct (38) of the magnetic resonance tomograph, and the drive thereof being made of non-ferromagnetic material, **characterized in that** the drive for pivoting the support (14, 17), via a control unit (32), being so controlled as to produce during the passive movement a series of pictures in real time within the magnetic resonance tomograph.

3. An apparatus according to claim 2, **characterized in that** the drive for pivoting the support (14, 17) is effected by at least one piezo-electromotor (22, 23).

4. An apparatus according to claims 2 or 3, **characterized in that** the control unit (32) is connected to ground and is shielded against magnetic radiation.

5. An apparatus according to claim 4, **characterized in that** the control unit (32) is disposed externally of the zone about the magnetic resonance tomogpaph wherein, during operation, the magnetic flux density is in excess of or equal to 0.2 tesla.

6. An apparatus according to any one of claims 4 or 5, **characterized in that** the control unit (32) is provided with at least one sensor, specifically with an optical encoder (21), for sensing the position of the support (14, 17).

7. An apparatus according to any one of claims 4 through 6, **characterized in that** the control unit (32) is connected to the drive (22, 23) of the support (14, 17) and, in the appropriate circumstances, to sensors (21) via grounded and shielded conduits (36, 37) which, externally of the duct (38) of the magnetic resonance tomograph, are provided with ferrites (31).

8. An apparatus according to any one of claims 2 through 7, **characterized in that** the support (14, 17) is pivotable about two axes (33, 34) in a manner independent of one another and driven by a motor.

9. An apparatus according to claim 8, **characterized in that** the support (14, 17) is pivotable about a first horizontal axis (33) and about a second axis (34) inclined over the vertical by about 35° in the horizontal plane and by about 18° in the sagittal plane.

10. An apparatus according to any one of claims 2 to 9, **characterized in that** means (18) are provided for fixing at least one part (19) of a patient's body to the support (14, 17), and that the support (19) at least in part is movable relative to the fixing means (18).

11. An apparatus according to claim 9, **characterized in that** the support (17) is pneumatically or hydraulically movable relative to the means (18) for fixing the part (19) of the patient's body.

12. An apparatus according to any one of claims 2 through 11, **characterized in that** the motorized drive of the support (14, 17) is in communication with the magnetic resonance tomograph, for example, via a control, such that pictures are taken by the magnetic resonance tomograph simultaneously with the generation of the passive movement of the part of a patient's body and/or of instruments or objects.

## Revendications

1. Procédé à photographier un mouvement passif d'un objet dans un tomographe à résonance magnétique comportant les mesures suivantes:
- fixer de l'objet sur an support (14, 17) ou des choses pareilles,
- produire un mouvement passif défini de l'objet par l'intermédiaire d'une commande motrice du support (14, 17) dedans le tube (38) d'un tomographe à résonance magnétique, et
- produire une série de prise photographique en temps réel pendant le mouvement de l'objet.

2. Appareil à produire d'un mouvement passif d'une partie du corps d'un patient et/ou des instruments ou objets compatibles aux tomographes à résonance magnétiques dedans un tomographe à résonance magnétique dans un procédé selon la revendication 1, comportant un support (14, 17) pour positionner au moins d'une partie du corps (19) d'un patient transportable en commun avec un lit du patient (35) ou des choses pareilles dans le tube (38) d'un tomographe à résonance magnétique, le support (14, 17) commandé par un moteur dedans le tube (38) du tomographe à résonance magnétique étant pivoté autour au moins d'un essieu (33, 34) et les components du support (14, 17) disposés dedans le tube (38) du tomographe à résonance magnétique et leur commande étant composés des matières non-ferromagnétiques, **caractérisé en ce que** le mécanisme à pivoter le support (14, 17) est commandé par l'intermédiaire d'un mécanisme à commande ou *control unit* (32) de sorte qu'une série de prises photographiques est produite en temps réel pendant le mouvement passif.

3. Appareil selon la revendication 2, **caractérisé en ce que** la commande à pivoter le le support (14, 17) se fait par l'intermédiaire au moins d'un piézo moteur électrique (22, 23).

4. Appareil selon les revendications 2 ou 3, **caractérisé en ce que** le mécanisme de commande ou *control unit* (32) est mis à terre et protégé contre la radiation magnétique.

5. Appareil selon la revendication 4, **caractérisé en ce que** le mécanisme de commande (32) est disposé à l'extérieure de la zone autour le tomographe à résonance magnétique dans laquelle la densité de flux magnétique, en action, est > ou = 0.2 tesla.

6. Appareil selon l'une des revendications 4 ou 5, **caractérisé en ce** le mécanisme de commande (32) est prévu au moins d'un palpeur, préférablement d' un mécanisme de codage (21) pour déterminer la position du support (14, 17).

7. Appareil selon l'une des revendications 4 à 6, **caractérisé en ce que** le mécanisme de commande est en communication avec le moteur (22, 23) du support (14, 17) et éventuellement avec les palpeurs (21) par l'intermédiaire des conduits (36, 37) mis en terre et protégés qui sont prévus, à l'extérieure du tube (38) du tomographe à résonance magnétique, des ferrites (31).

8. Appareil selon l'une des revendications 2 à 7, **caractérisé en ce que** le support (14, 17) est pivotable autour de deux essieux (33, 34) commandées par un moteur de manière indépendante l'un de l'autre.

9. Appareil selon la revendication 8, **caractérisé en ce que** le support (14, 17) est pivotable autour d'un essieu (33) premier horizontale et autour d'un essieu (34) secondaire incliné vis à vis la verticale par environ 35° dans le plan horizontale et par environ 18° dans le plan sagittal.

10. Appareil selon l'une des revendications 2 à 9, **caractérisé en ce que** des moyens (18) à fixer au moins d'une partie du corps (19) d'un patient sur un support (14, 17) sont prévus, et que le support (17) est mouvant au moins dans des certaines zones relatif au moyens (18) à fixer.

11. Appareil selon la revendication 9, **caractérisé en ce que** le support (17) est mobile de manière pneumatique ou hydraulique relatif aux moyens (18) à fixer la partie du corps (19).

12. Appareil selon l'une des revendications 2 à 11, **caractérisé en ce que** la commande motrice du support (14, 17) est en communication, par exemple, par l'intermédiaire d'une commande, avec le tomographe à résonance magnétique de sorte que des photographies sont prises par le tomographe à résonance magnétique en même temps avec la production du mouvement passif d'une partie de corps d'un patient et/ou des instruments ou objets.
